# EUROPÄISCHE PATENTSCHRIFT

(11) **EP 2 182 991 B1**
(45) Veröffentlichungstag und Bekanntmachung des Hinweises auf die Patenterteilung: **04.09.2013**
(21) Anmeldenummer: 08784352.0
(22) Anmeldetag: 15.07.2008
(51) Int. Cl.: A61L 2/14

(54) **VERFAHREN UND VORRICHTUNG ZUM STERILISIEREN SOWIE VORRICHTUNG ZUR BLASFORMUNG VON VORFORMLINGEN**
METHOD AND DEVICE FOR STERILIZATION AND DEVICE FOR BLOW MOULDING OF PREFORMS
PROCÉDÉ ET DISPOSITIF DE STÉRILISATION ET DISPOSITIF DE FORMAGE PAR SOUFFLAGE DE PRÉFORMES

(30) Priorität: 01.09.2007 DE 102007041573
(43) Veröffentlichungstag der Anmeldung: 12.05.2010
(73) Patentinhaber: KHS Corpoplast GmbH, 22145 Hamburg (DE)
(72) Erfinder: HAESENDONCKX, Frank, 22395 Hamburg (DE); VOGEL, Klaus, 22885 Barsbüttel (DE); HOFFMANN, Werner, 20259 Hamburg (DE)
(74) Vertreter: Hausfeld, Norbert
(86) Internationale Anmeldenummer: PCT/DE2008/001171
(87) Internationale Veröffentlichungsnummer: WO 2009/026869

(56) Entgegenhaltungen:
- EP-A1- 0 976 527
- WO-A-99/17334
- WO-A-2004/024577
- DE-A1- 19 502 103
- GB-A- 2 290 221
- US-A- 5 798 139

## Beschreibung

Die Erfindung betrifft ein Verfahren zum Sterilisieren von spritzgegossenen vorformlingen aus einem thermoplastischen Material, die zur Herstellung von blasgeformten Behältern vorgesehen sind.

Darüber hinaus betrifft die Erfindung ein verfahren zum Blasformen von mindestens bereichsweise sterilen Behältern.

Schließlich betrifft die Erfindung eine Vorrichtung zum Sterilisieren von Vorformlingen aus einem thermoplastischen Material, die zur Herstellung von blasgeformten Behältern vorgesehen sind. Ebenfalls ist von der Erfindung eine Vorrichtung zur Herstellung von mindestens bereichsweise sterilen Behältern aus einem thermoplastischen Material umfaßt.

Die Erfindung betrifft auch eine Vorrichtung zur Blasformung von Behältern, die mindestens eine auf einer Tragstruktur angeordnete Blasstation zur Umformung von spritzgegossenen thermoplastischen Vorformlingen in die Behälter aufweist.

Eine Herstellung von sterilen blasgeformten Behältern erfolgt typischerweise derart, daß diese Behälter nach ihrer Blasformung und vor einer Befüllung unter Verwendung von Wasserstoffperoxid oder anderen Chemikalien sterilisiert werden. Ebenfalls ist es bereits bekannt, die bei der Blasformung der Behälter als Ausgangsprodukt verwendeten Vorformlinge zu sterilisieren, insbesondere den Bereich der inneren Oberfläche dieser Vorformlinge.

Es ist ebenfalls bereits bekannt, daß bei der Durchführung einer Beschichtung von Behältern unter Anwendung eines Plasmas Sterilisierungseffekte auftreten. Aufgrund der üblicherweise vorgesehenen Konturen im Bereich der Behälterwandungen treten bei derartigen sterilisierungen aber sogenannten Abschattungen auf, die einer zuverlässigen Sterilisierung entgegenstehen.

In der DE 195 02 103 A1, der US 5,798,139 A sowie der WO 99/17334 A werden jeweils derartige Beschichtungen von zuvor blasgeformten Behältern beschrieben, bei denen als Folge der Erzeugung der Beschichtungen sterile Oberflächen der beschichteten Behälter bereitgestellt werden.

Bei einer Behälterformung durch Blasdruckeinwirkung werden vorformlinge aus einem thermoplastischen Material, beispielsweise Vorformlinge aus PET (Polyethylenterephtalat), innerhalb einer Blasmaschine unterschiedlichen Bearbeitungsstationen zugeführt. Typischerweise weist eine derartige Blasmaschine eine Heizeinrichtung sowie eine Blaseinrichtung auf, in deren Bereich der zuvor temperierte Vorformling durch biaxiale Orientierung zu einem Behälter expandiert wird. Die Expansion erfolgt mit Hilfe von Druckluft, die in den zu expandierenden Vorformling eingeleitet wird. Der verfahrenstechnische Ablauf bei einer derartigen Expansion des Vorformlings wird in der DE-OS 43 40 291 erläutert.

Der grundsätzliche Aufbau einer Blasstation zur Behälterformung wird in der DE-OS 42 12 583 beschrieben. Möglichkeiten zur Temperierung der Vorformlinge werden in der DE-OS 23 52 926 erläutert.

Innerhalb der Vorrichtung zur Blasformung können die Vorformlinge sowie die geblasenen Behälter mit Hilfe unterschiedlicher Handhabungseinrichtungen transportiert werden. Bewährt hat sich insbesondere die Verwendung von Transportdornen, auf die die Vorformlinge aufgesteckt werden. Die Vorformlinge können aber auch mit anderen Trageinrichtungen gehandhabt werden. Die verwendung von Greifzangen zur Handhabung von vorformlingen und die Verwendung von Spreizdornen, die zur Halterung in einen Mündungsbereich des Vorformlings einführbar sind, gehören ebenfalls zu den verfügbaren Konstruktionen.

Eine Handhabung von Behältern unter Verwendung von Übergaberädern wird beispielsweise in der DE-OS 199 06 438 bei einer Anordnung des Übergaberades zwischen einem Blasrad und einer Ausgabestrecke beschrieben.

Die bereits erläuterte Handhabung der Vorformlinge erfolgt zum einen bei den sogenannten Zweistufenverfahren, bei denen die Vorformlinge zunächst in einem Spritzgußverfahren hergestellt, anschließend zwischengelagert und erst später hinsichtlich ihrer Temperatur konditioniert und zu einem Behälter aufgeblasen werden. Zum anderen erfolgt eine Anwendung bei den sogenannten Einstufenverfahren, bei denen die Vorformlinge unmittelbar nach ihrer spritzgußtechnischen Herstellung und einer ausreichenden Verfestigung geeignet temperiert und anschließend aufgeblasen werden.

Im Hinblick auf die verwendeten Blasstationen sind unterschiedliche Ausführungsformen bekannt. Bei Blasstationen, die auf rotierenden Transporträdern angeordnet sind, ist eine buchartige Aufklappbarkeit der Formträger häufig anzutreffen. Es ist aber auch möglich, relativ zueinander verschiebliche oder andersartig geführte Formträger einzusetzen. Bei ortsfesten Blasstationen, die insbesondere dafür geeignet sind, mehrere Kavitäten zur Behälterformung aufzunehmen, werden typischerweise parallel zueinander angeordnete Platten als Formträger verwendet.

Aufgabe der vorliegenden Erfindung ist es, ein Verfahren der einleitend genannten Art derart zu verbessern, daß in einfacher Weise eine zuverlässige Sterilisation durchgeführt werden kann.

Diese Aufgabe wird erfindungsgemäß dadurch gelöst, daß die Sterilisierung durch Anwendung eines Plasmas durchgeführt wird und daß die Vorformlinge nach der Sterilisierung der Blasformung zugeführt werden.

Weitere Aufgabe der vorliegenden Erfindung ist es, eine Vorrichtung der einleitend genannten Art derart zu konstruieren, daß eine effektive Sterilisation mit geringem Aufwand durchführbar ist.

Diese Aufgabe wird erfindungsgemäß dadurch gelöst, daß mindestens eine Sterilisiereinheit als ein Plasmagenerator ausgebildet ist und daß die vorformlinge nach der Sterilisierung der Blasformung zugeführt werden. Schließlich besteht eine Aufgabe der vorliegenden Erfindung darin, eine Vorrichtung zur Blasformung von Behältern der einleitend genannten Art derart zu konstruieren, daß die Herstellung von sterilen Behältern unterstützt wird.

Diese Aufgabe wird erfindungsgemäß dadurch gelöst, daß entlang eines Teiles eines Transportweges der Vorformlinge eine Sterilisiereinheit angeordnet ist, die mit einen Plasmagenerator versehen ist.

Für eine Vielzahl von Anwendungen ist es ausreichend, daß eine innere Oberfläche der Vorformlinge sterilisiert wird.

Darüber hinaus ist aber auch daran gedacht, daß eine äußere Oberfläche der Vorformlinge sterilisiert wird.

Eine preiswerte Verfahrensdurchführung wird dadurch unterstützt, daß die Plasmabehandlung bei Umgebungsdruck durchgeführt wird.

Eine Intensivierung der Plasmaeinwirkung ist dadurch möglich, daß die Plasmabehandlung bei einem gegenüber der Umgebung erhöhten Druck durchgeführt wird.

Eine Beeinflussung des generierten Plasmas kann dadurch erfolgen, daß die Plasmabehandlung bei Anwendung eines Inertgases durchgeführt wird.

Optimale Bedingungen für die Durchführung der Sterilisierung werden dadurch erreicht, daß die Plasmabehandlung bei einer Gasfeuchtigkeit in einem Bereich von 15 bis 95% relativ zu einer maximalen Feuchtigkeitsaufnahme durchgeführt wird.

Eine typische Sterilisationszeit wird dadurch bereitgestellt, daß die Plasmabehandlung für einen Zeitraum von einer bis zehn Sekunden durchgeführt wird.

Eine Generierung des Plasmas in räumlicher Nähe zur inneren Oberfläche des Vorformlings wird dadurch ermöglicht, daß die Plasmabehandlung unter Verwendung einer Hochspannungselektrode durchgeführt wird, die in einem Innenraum des Vorformlings positioniert wird.

Eine gleichmäßige Einwirkung des Plasmas bei geringem Energieeinsatz wird dadurch unterstützt, daß die Elektrode mit einem Abstand von 0,1 bis 3 mm relativ zu einer Innenseite des Vorformlings positioniert wird.

Eine gleichmäßige Ausbreitung des Plasmas wird auch dadurch unterstützt, daß der Vorformling mindestens bereichsweise von einer Außenelektrode während der Durchführung der Plasmabehandlung umgeben wird.

Eine optimale Feldverteilung zwischen den Elektroden wird dadurch unterstützt, daß die Außenelektrode mit einem Abstand von 0 bis 2 mm zu einer Außenseite des Vorformlings positioniert wird.

Möglichkeiten zur Steuerung des Plasmas werden dadurch bereitgestellt, daß das Plasma durch einen Hochspannungsgenerator mit einer gepulsten variablen Frequenz generiert wird.

Eine Durchführung der Sterilisierung als Teil eines Transportvorganges der Vorformlinge wird dadurch unterstützt, daß der Vorformling gemeinsam mit der Sterilisiereinrichtung entlang eines Transportweges bewegt wird.

In den Zeichnungen sind Ausführungsbeispiele der Erfindung schematisch dargestellt. Es zeigen:
- Fig. 1: Eine perspektivische Darstellung einer Blasstation zur Herstellung von Behältern aus Vorformlingen,
- Fig. 2: einen Längsschnitt durch eine Blasform, in der ein Vorformling gereckt und expandiert wird,
- Fig. 3: eine Skizze zur veranschaulichung eines grundsätzlichen Aufbaus einer Vorrichtung zur Blasformung von Behältern,
- Fig. 4: eine modifizierte Heizstrecke mit vergrößerter Heizkapazität,
- Fig. 5: einen Längsschnitt durch einen Vorformling, der in eine Plasmabehandlungseinrichtung eingesetzt ist und
- Fig. 6: einen Querschnitt gemäß Schnittlinie VI-VI in Fig. 5.

Vor einer Erläuterung des Detailaufbaus der Vorrichtung zum Sterilisieren der Vorformlinge (1) durch Anwendung eines Plasmas sowie vor einer Erläuterung eines konkreten Einbaues einer entsprechenden Vorrichtung in eine Blasmaschine soll nachfolgend zunächst der grundsätzliche Aufbau einer Blasmaschine beschrieben werden.

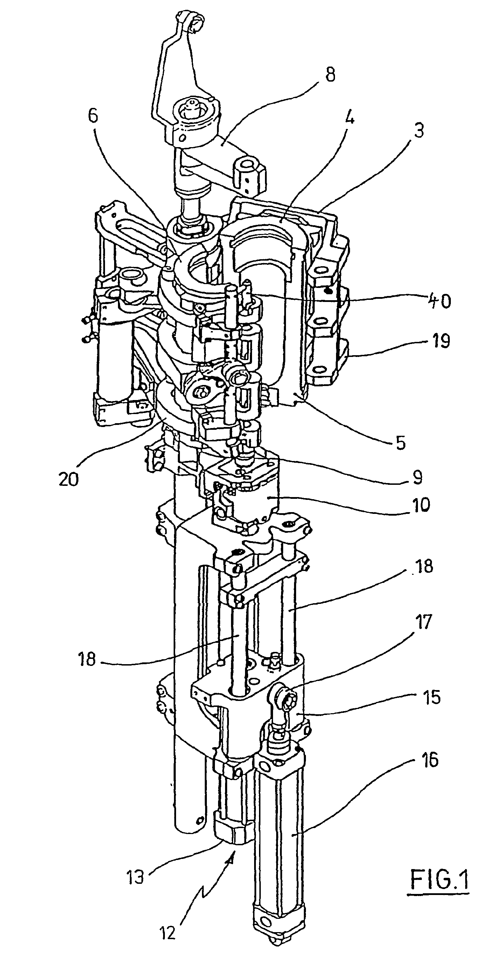

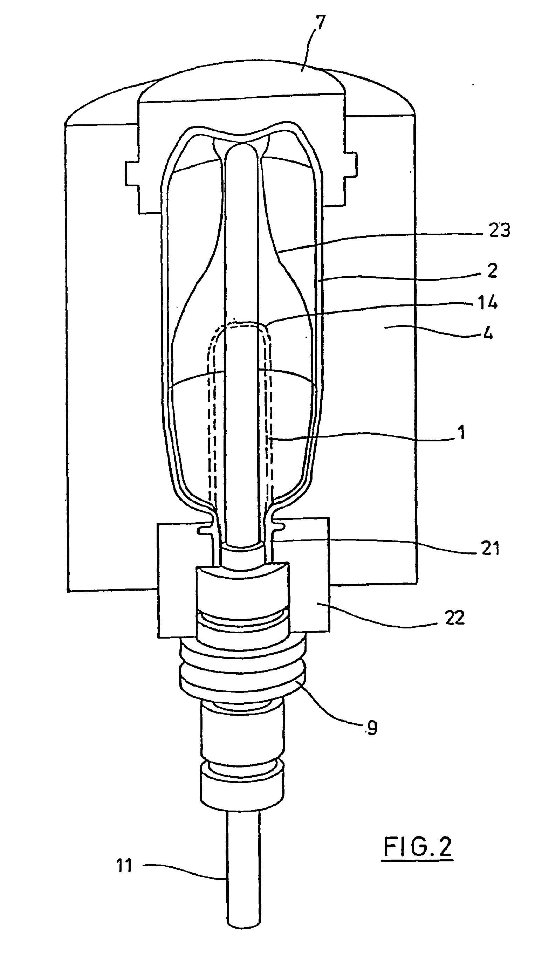

Der prinzipielle Aufbau einer Vorrichtung zur Umformung von Vorformlingen (1) in Behälter (2) ist in Fig. 1 und in Fig. 2 dargestellt.

Die Vorrichtung zur Formung des Behälters (2) besteht im wesentlichen aus einer Blasstation (3), die mit einer Blasform (4) versehen ist, in die ein Vorformling (1) einsetzbar ist. Der Vorformling (1) kann ein spritzgegossenes Teil aus Polyethylenterephthalat sein. Zur Ermöglichung eines Einsetzens des Vorformlings (1) in die Blasform (4) und zur Ermöglichung eines Herausnehmens des fertigen Behälters (2) besteht die Blasform (4) aus Formhälften (5, 6) und einem Bodenteil (7), das von einer Hubvorrichtung (8) positionierbar ist. Der Vorformling (1) kann im Bereich der Blasstation (3) von einem Transportdorn (9) gehalten sein, der gemeinsam mit dem Vorformling (1) eine Mehrzahl von Behandlungsstationen innerhalb der Vorrichtung durchläuft. Es ist aber auch möglich, den Vorformling (1) beispielsweise über Zangen oder andere Handhabungsmittel direkt in die Blasform (4) einzusetzen.

Zur Ermöglichung einer Druckluftzuleitung ist unterhalb des Transportdornes (9) ein Anschlußkolben (10) angeordnet, der dem Vorformling (1) Druckluft zuführt und gleichzeitig eine Abdichtung relativ zum Transportdorn (9) vornimmt. Bei einer abgewandelten Konstruktion ist es grundsätzlich aber auch denkbar, feste Druckluftzuleitungen zu verwenden.

Eine Reckung des Vorformlings (1) erfolgt mit Hilfe einer Reckstange (11), die von einem Zylinder (12) positioniert wird. Grundsätzlich ist es aber auch denkbar, eine mechanische Positionierung der Reckstange (11) über Kurvensegmente durchzuführen, die von Abgriffrollen beaufschlagt sind. Die Verwendung von Kurvensegmenten ist insbesondere dann zweckmäßig, wenn eine Mehrzahl von Blasstationen (3) auf einem rotierenden Blasrad angeordnet sind. Eine Verwendung von Zylindern (12) ist zweckmäßig, wenn ortsfest angeordnete Blasstationen (3) vorgesehen sind.

Bei der in Fig. 1 dargestellten Ausführungsform ist das Recksystem derart ausgebildet, daß eine Tandem-Anordnung von zwei Zylindern (12) bereitgestellt ist. Von einem Primärzylinder (13) wird die Reckstange (11) zunächst vor Beginn des eigentlichen Reckvorganges bis in den Bereich eines Bodens (14) des Vorformlings (1) gefahren. Während des eigentlichen Reckvorganges wird der Primärzylinder (13) mit ausgefahrener Reckstange gemeinsam mit einem den Primärzylinder (13) tragenden Schlitten (15) von einem Sekundärzylinder (16) oder über eine Kurvensteuerung positioniert. Insbesondere ist daran gedacht, den Sekundärzylinder (16) derart kurvengesteuert einzusetzen, daß von einer Führungsrolle (17), die während der Durchführung des Reckvorganges an einer Kurvenbahn entlang gleitet, eine aktuelle Reckposition vorgegeben wird. Die Führungsrolle (17) wird vom Sekundärzylinder (16) gegen die Führungsbahn gedrückt. Der Schlitten (15) gleitet entlang von zwei Führungselementen (18).

Nach einem Schließen der im Bereich von Trägern (19, 20) angeordneten Formhälften (5, 6) erfolgt eine Verriegelung der Träger (19, 20) relativ zueinander mit Hilfe einer Verriegelungseinrichtung (40).

Zur Anpassung an unterschiedliche Formen eines Mündungsabschnittes (21) des Vorformlings (1) ist gemäß Fig. 2 die Verwendung separater Gewindeeinsätze (22) im Bereich der Blasform (4) vorgesehen.

Fig. 2 zeigt zusätzlich zum geblasenen Behälter (2) auch gestrichelt eingezeichnet den Vorformling (1) und schematisch eine sich entwickelnde Behälterblase (23).

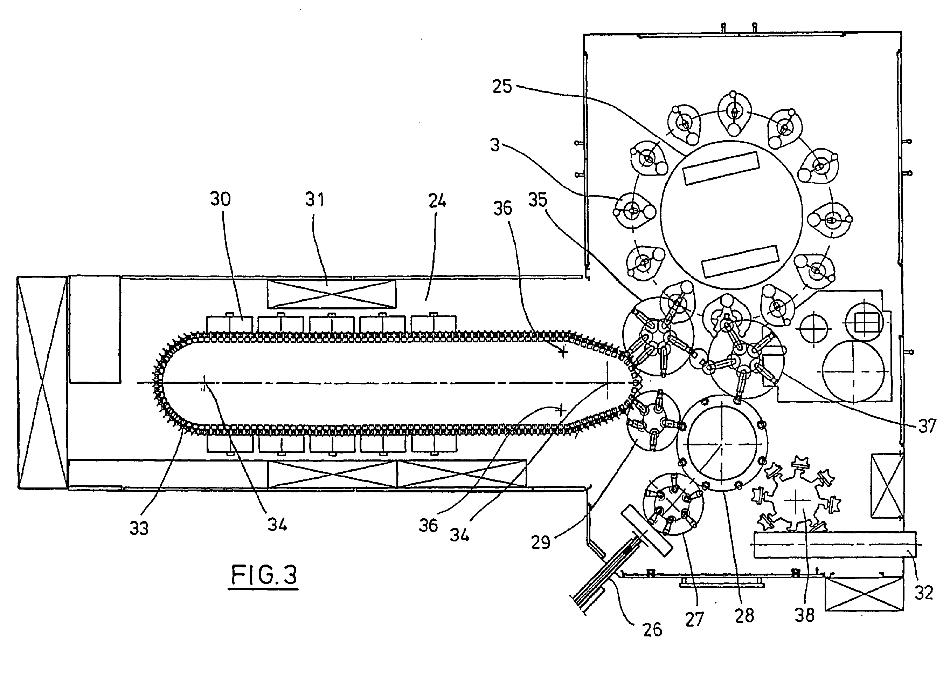

Fig. 3 zeigt den grundsätzlichen Aufbau einer Blasmaschine, die mit einer Heizstrecke (24) sowie einem rotierenden Blasrad (25) versehen ist. Ausgehend von einer Vorformlingseingabe (26) werden die Vorformlinge (1) von Übergaberädern (27, 28, 29) in den Bereich der Heizstrecke (24) transportiert. Entlang der Heizstrecke (24) sind Heizstrahler (30) sowie Gebläse (31) angeordnet, um die Vorformlinge (1) zu temperieren. Nach einer ausreichenden Temperierung der Vorformlinge (1) werden diese an das Blasrad (25) übergeben, in dessen Bereich die Blasstationen (3) angeordnet sind. Die fertig geblasenen Behälter (2) werden von weiteren Übergaberädern einer Ausgabestrecke (32) zugeführt.

Um einen Vorformling (1) derart in einen Behälter (2) umformen zu können, daß der Behälter (2) Materialeigenschaften aufweist, die eine lange Verwendungsfähigkeit von innerhalb des Behälters (2) abgefüllten Lebensmitteln, insbesondere von Getränken, gewährleisten, müssen spezielle Verfahrensschritte bei der Beheizung und Orientierung der Vorformlinge (1) eingehalten werden. Darüber hinaus können vorteilhafte Wirkungen durch Einhaltung spezieller Dimensionierungsvorschriften erzielt werden.

Als thermoplastisches Material können unterschiedliche Kunststoffe verwendet werden. Einsatzfähig sind beispielsweise PET, PEN oder PP.

Die Expansion des Vorformlings (1) während des Orientierungsvorganges erfolgt durch Druckluftzuführung. Die Druckluftzuführung ist in eine Vorblasphase, in der Gas, zum Beispiel Preßluft, mit einem niedrigen Druckniveau zugeführt wird und in eine sich anschließende Hauptblasphase unterteilt, in der Gas mit einem höheren Druckniveau zugeführt wird. während der Vorblasphase wird typischerweise Druckluft mit einem Druck im Intervall von 10 bar bis 25 bar verwendet und während der Hauptblasphase wird Druckluft mit einem Druck im Intervall von 25 bar bis 40 bar zugeführt.

Aus Fig. 3 ist ebenfalls erkennbar, daß bei der dargestellten Ausführungsform die Heizstrecke (24) aus einer Vielzahl umlaufender Transportelemente (33) ausgebildet ist, die kettenartig aneinandergereiht und entlang von Umlenkrädern (34) geführt sind. Insbesondere ist daran gedacht, durch die kettenartige Anordnung eine im wesentlichen rechteckförmige Grundkontur aufzuspannen. Bei der dargestellten Ausführungsform werden im Bereich der dem Übergaberad (29) und einem Eingaberad (35) zugewandten Ausdehnung der Heizstrecke (24) ein einzelnes relativ groß dimensioniertes Umlenkrad (34) und im Bereich von benachbarten Umlenkungen zwei vergleichsweise kleiner dimensionierte Umlenkräder (36) verwendet. Grundsätzlich sind aber auch beliebige andere Führungen denkbar.

Zur Ermöglichung einer möglichst dichten Anordnung des Übergaberades (29) und des Eingaberades (35) relativ zueinander erweist sich die dargestellte Anordnung als besonders zweckmäßig, da im Bereich der entsprechenden Ausdehnung der Heizstrecke (24) drei Umlenkräder (34, 36) positioniert sind, und zwar jeweils die kleineren Umlenkräder (36) im Bereich der Überleitung zu den linearen Verläufen der Heizstrecke (24) und das größere Umlenkrad (34) im unmittelbaren Übergabebereich zum Übergaberad (29) und zum Eingaberad (35). Alternativ zur Verwendung von kettenartigen Transportelementen (33) ist es beispielsweise auch möglich, ein rotierendes Heizrad zu verwenden.

Nach einem fertigen Blasen der Behälter (2) werden diese von einem Entnahmerad (37) aus dem Bereich der Blasstationen (3) herausgeführt und über das Übergaberad (28) und ein Ausgaberad (38) zur Ausgabestrecke (32) transportiert.

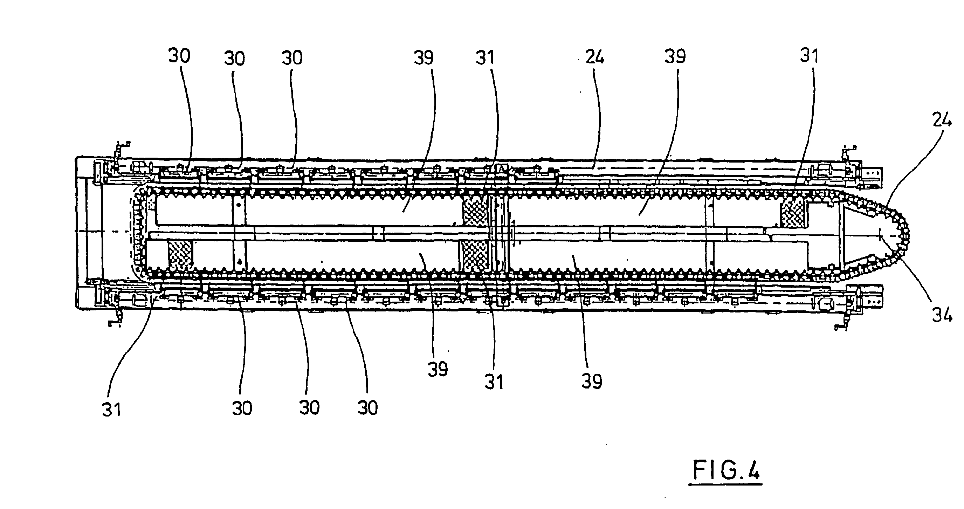

In der in Fig. 4 dargestellten modifizierten Heizstrekke (24) können durch die größere Anzahl von Heizstrahlern (30) eine größere Menge von Vorformlingen (1) je Zeiteinheit temperiert werden. Die Gebläse (31) leiten hier Kühlluft in den Bereich von Kühlluftkanälen (39) ein, die den zugeordneten Heizstrahlern (30) jeweils gegenüberliegen und über Ausströmöffnungen die Kühlluft abgeben. Durch die Anordnung der Ausströmrichtungen wird eine Strömungsrichtung für die Kühlluft im wesentlichen quer zu einer Transportrichtung der Vorformlinge (1) realisiert. Die Kühlluftkanäle (39) können im Bereich von den Heizstrahlern (30) gegenüberliegenden Oberflächen Reflektoren für die Heizstrahlung bereitstellen, ebenfalls ist es möglich, über die abgegebene Kühlluft auch eine Kühlung der Heizstrahler (30) zu realisieren.

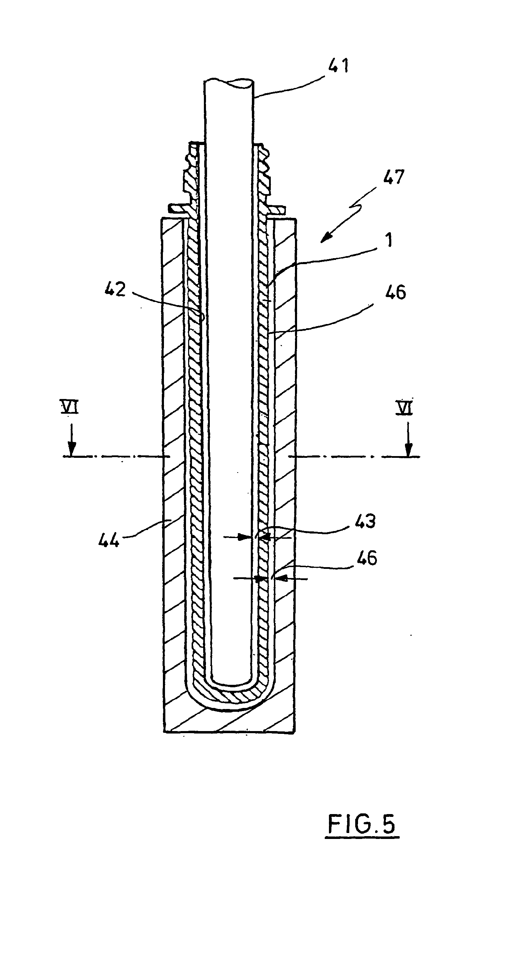

Fig. 5 zeigt einen Längsschnitt durch einen Vorformling (1), in den eine Stabelektrode (41) eingeführt ist. Zwischen der Stabelektrode (41) und einer Innenwand (42) des Vorformlings (1) erstreckt sich ein Abstand (43) von etwa 0,1 bis 3,0 mm.

Der Vorformling (1) ist mindestens bereichsweise in eine hülsenartige Außenelektrode (44) eingeführt, die einen Abstand (45) zu einer Außenseite (46) des Vorformlings (1) in einem Bereich von 0 bis 2 mm aufweist.

Typischerweise wird die Außenelektrode (44) elektrisch auf Masse gelegt und an die Stabelektrode (41) wird eine Hochspannung angelegt. Ausgehend von der Stabelektrode (41) wird durch die erläuterte Anordnung eine Plasmaentladung generiert, die auf die Innenseite (42) des Vorformlings (1) einwirkt und hier Bakterien sowie Keime zersetzt. Gemäß einem Ausführungsbeispiel erfolgt die Plasmaentladung bei Normaldruck. Es ist ebenfalls daran gedacht, Plasmaentladungen bei vermindertem oder erhöhtem Druck durchzuführen. Gegebenenfalls wird die Luft innerhalb des Vorformlings (1) getrocknet oder befeuchtet. Es können hierdurch Feuchtigkeitswerte in einem Bereich von 15 bis 95% eingestellt werden. Ebenfalls ist an eine Zuführung spezieller Gase gedacht.

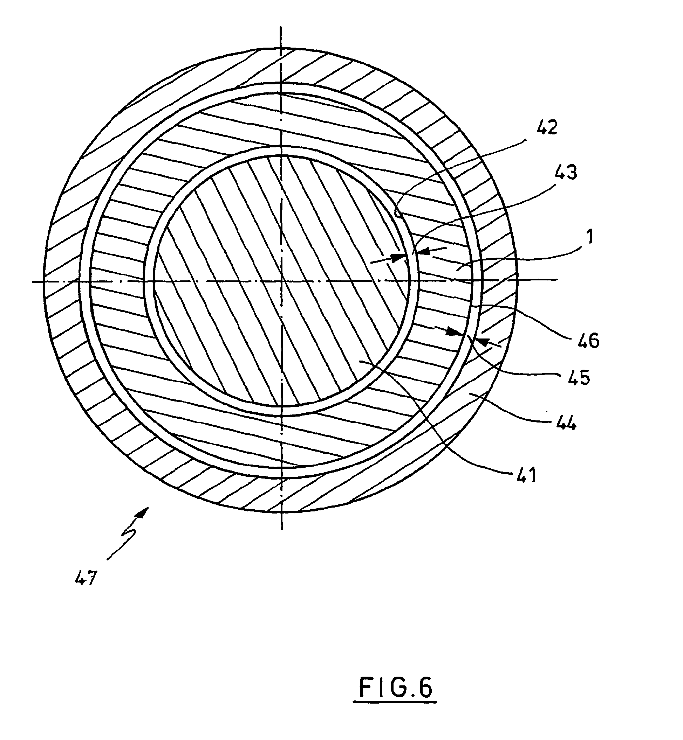

Fig. 6 zeigt einen Querschnitt durch die Anordnung gemäß Fig. 5. Es ist hierdurch im wesentlichen die koaxiale Anordnung der Stabelektrode (41), des Vorformlings (1) und der Außenelektrode (44) relativ zueinander erkennbar. Die Stabelektrode (41) kann wie im dargestellten Ausführungsbeispiel als ein massiver Stab ausgebildet sein, grundsätzlich ist jedoch eine rohrförmige Gestaltung ausreichend.

Gemäß einem weiteren Ausführungsbeispiel wird eine Sterilisiereinrichtung (47) verwendet, bei der das Plasma nicht innerhalb des Vorformlings (1) generiert, sondern dem Innenraum des Vorformlings (1) zugeführt wird. Beispielsweise ist es möglich, einen sogenannten Plasma-Jet zu verwenden und das ionisierte Gas durch eine rohrförmige Anordnung bis in einen Bodenbereich des Vorformlings (1) zu leiten und durch den Abstand (43) ausströmen zu lassen. Alternativ kann ein entsprechendes Rohr, das ähnlich wie die Stabelektrode (41) in Fig. 5 angeordnet ist, auch mit seitlichen Ausströmöffnungen versehen sein.

Eine Anordnung der Sterilisiereinrichtung (47) erfolgt beispielsweise im Bereich der Blasmaschine in Transportrichtung der Vorformlinge (1) vor der Heizstrecke (24). Insbesondere ist daran gedacht, die Sterilisiereinrichtung (47) mit entlang eines Transportweges umlaufend angeordneten Elektroden (41, 44) zu versehen. Die Plasmabehandlung der Vorformlinge (1) kann hierdurch in einem kontinuierlichen Prozeß erfolgen. Beispielsweise ist es möglich, die Vorformlinge (1) in die Außenelektrode (44) einzuführen und die Stabelektrode (41) in den Vorformling (1) hinein zu fahren. Bei einer derartigen Ausführungsform würde sich der Vorformling (1) auf einem konstanten Höhenniveau bewegen und die Elektroden (41, 44) sind relativ zum Vorformling (1) beweglich angeordnet.

Es ist aber ebenfalls denkbar, den Vorformling (1) beweglich relativ zu mindestens einer der Elektroden (41, 44) anzuordnen. Ebenfalls ist daran gedacht, den Vorformling (1) zunächst mit einer der Elektroden (41, 44) zu kombinieren und anschließend diese Anordnung gemeinsam relativ zur verbleibenden Elektrode (41, 44) zu positionieren.

Schließlich ist es gemäß einem weiteren Ausführungsbeispiel möglich, die Außenelektrode (44) in Richtung einer Längsachse des Vorformlings (1) zu teilen und eine Querbeweglichkeit relativ zum Vorformling zu realisieren.

Bei einer externen Erzeugung des Plasmas ist auch daran gedacht, den Vorformling sequentiell an einer Mehrzahl von Plasmadüsen vorbeizuführen, die jeweils einen Plasmastrom in den Innenraum des Vorformlings (1) einleiten.

Gemäß einer bereits erwähnten bevorzugten Ausführungsform der vorliegenden Erfindung ist insbesondere daran gedacht, die Plasmaentladung zur Durchführung der Sterilisation nicht in einem Vakuum oder bei stark vermindertem Luftdruck durchzuführen, sondern das Sterilisationsverfahren bei Normaldruck, und insbesondere bei einer Normalatmosphäre zu realisieren. Ebenfalls ist für eine Intensivierung der sterilisierenden Wirkung der Plasmaentladung die Anwendung von Überdruck vorteilhaft. Die Wirkung der Sterilisierung läßt sich durch die Gasfeuchtigkeit beeinflussen, wobei insbesondere eine erhöhte Gasfeuchtigkeit als vorteilhaft angesehen wird. Als besonders vorteilhaft erweist sich die Kombination einer Plasmaentladung mit einem Druck oberhalb der Normalatmosphäre und mit einer hohen Gasfeuchtigkeit.

## Patentansprüche

1. Verfahren zum Sterilisieren von spritzgegossenen Vorformlingen (1) aus einem thermoplastischen Material, die zur Herstellung von blasgeformten Behältern (2) vorgesehen sind, **dadurch gekennzeichnet, daß** die Sterilisierung durch Anwendung eines Plasmas durchgeführt wird und daß die Vorformlinge (2) nach der Sterilisierung der Blasformung zugeführt werden.

2. verfahren nach Anspruch 1, **dadurch gekennzeichnet, daß** eine innere Oberfläche der Vorformlinge (1) sterilisiert wird.

3. Verfahren nach Anspruch 1, **dadurch gekennzeichnet, daß** eine äußere Oberfläche der Vorformlinge (1) sterilisiert wird.

4. Verfahren nach einem der Ansprüche 1 bis 3, **dadurch gekennzeichnet, daß** die Plasmabehandlung bei Umgebungsdruck durchgeführt wird.

5. verfahren nach einem der Ansprüche 1 bis 3, **dadurch gekennzeichnet, daß** die Plasmabehandlung bei einem gegenüber der Umgebung erhöhten Druck durchgeführt wird.

6. Verfahren nach einem der Ansprüche 1 bis 5, **dadurch gekennzeichnet, daß** die Plasmabehandlung bei Anwendung eines Inertgases durchgeführt wird.

7. Verfahren nach einem der Ansprüche 1 bis 6, **dadurch gekennzeichnet, daß** die Plasmabehandlung bei einer Gasfeuchtigkeit in einem Bereich von 15 bis 95% relativ zu einer maximalen Feuchtigkeitsaufnahme durchgeführt wird.

8. Verfahren nach einem der Ansprüche 1 bis 7, **dadurch gekennzeichnet, daß** die Plasmabehandlung für einen Zeitraum von einer bis zehn Sekunden durchgeführt wird.

9. Verfahren nach einem der Ansprüche 1 bis 8, **dadurch gekennzeichnet, daß** die Plasmabehandlung unter Verwendung einer Hochspannungselektrode (41) durchgeführt wird, die in einem Innenraum des Vorformlings (1) positioniert wird.

10. Verfahren nach Anspruch 9, **dadurch gekennzeichnet, daß** die Elektrode (41) mit einem Abstand von 0,1 bis 3 mm relativ zu einer Innenseite (42) des Vorformlings (1) positioniert wird.

11. Verfahren nach einem der Ansprüche 1 bis 10, **dadurch gekennzeichnet, daß** der Vorformling (1) mindestens bereichsweise von einer Außenelektrode (44) während der Durchführung der Plasmabehandlung umgeben wird.

12. Verfahren nach Anspruch 11, **dadurch gekennzeichnet, daß** die Außenelektrode (44) mit einem Abstand von 0 bis 2 mm zu einer Außenseite (46) des Vorformlings (1) positioniert wird.

13. Verfahren nach einem der Ansprüche 1 bis 12, **dadurch gekennzeichnet, daß** das Plasma durch einen Hochspannungsgenerator mit einer gepulsten Variablen Frequenz generiert wird.

14. Verfahren nach einem der Ansprüche 1 bis 13, **dadurch gekennzeichnet, daß** der Vorformling (1) gemeinsam mit der Sterilisiereinrichtung (47) entlang eines Transportweges bewegt wird.

15. Vorrichtung zum Sterilisieren von spritzgegossenen vorformlingen aus einem thermoplastischen Material, die zur Herstellung von blasgeformten Behältern vorgesehen sind, **dadurch gekennzeichnet, daß** mindestens eine Sterilisiereinrichtung (47) mit einem Plasmagenerator versehen ist und daß die Vorformlinge (2) nach der Sterilisierung der Blasformung zugeführt werden.

16. Vorrichtung nach Anspruch 15, **dadurch gekennzeichnet, daß** der Plasmagenerator eine Stabelektrode (41) zum Einführen in einen Innenraum des vorformlings (1) aufweist.

17. Vorrichtung nach Anspruch 15 oder 16, **dadurch gekennzeichnet, daß** der Plasmagenerator eine Außenelektrode (44) zur mindestens bereichsweisen Umschließung des Vorformlings (1) aufweist.

18. Vorrichtung nach Anspruch 17, **dadurch gekennzeichnet, daß** die Außenelektrode (44) becherartig ausgebildet ist.

19. Vorrichtung nach einem der Ansprüche 15 bis 18, **dadurch gekennzeichnet, daß** die Innenelektrode mit einem Abstand von 0,1 bis 3 mm zu einer Innenseite (42) des Vorformlings (1) angeordnet ist.

20. Vorrichtung zur Blasformung von Behältern (2), die mindestens eine auf einer Tragstruktur angeordnete Blasstation (3) zur Umformung von thermoplastischen spritzgegossenen Vorformlingen (1) in die Behälter (2) aufweist, **dadurch gekennzeichnet, daß** entlang eines Transportweges der Vorformlinge (1) mindestens eine mit einem Plasmagenerator versehene Sterilisiereinrichtung (47) angeordnet ist und daß die Sterilisiereinrichtung (47) entlang
des Transportweges vor der Blasstation (3) angeordnet ist.

## Claims

1. A method for the sterilisation of injection moulded preforms (1) made of a thermoplastic material, said preforms (1) being provided for the manufacture of blow moulded containers (2),
**characterised in that**
- the sterilisation is carried out by applying a plasma and that the preforms (2) are supplied to the blow moulding station after sterilisation.

2. The method according to Claim 1,
**characterised in that**
- an internal surface of the preforms (1) is being sterilised.

3. The method according to Claim 1,
**characterised in that**
- an external surface of the preform (1) is being sterilised.

4. The method according to any one of Claims 1 to 3,
**characterised in that**
- the plasma treatment is carried out at ambient pressure.

5. The method according to any one of Claims 1 to 3,
**characterised in that**
- the plasma treatment is carried out at a pressure that is increased as compared to the environment.

6. The method according to any one of Claims 1 to 5,
**characterised in that**
- the plasma treatment is carried out under application of an inert gas.

7. The method according to any one of Claims 1 to 6,
**characterised in that**
- the plasma treatment is carried out at a gas humidity within a range from 15 to 95% in relation to a maximum absorption of humidity.

8. The method according to any one of Claims 1 to 7,
**characterised in that**
- the plasma treatment is carried out for a time period ranging from one to ten seconds.

9. The method according to any one of Claims 1 to 8,
**characterised in that**
- the plasma treatment is carried out under application of a high voltage electrode (41) which is positioned in an interior region of the preform (1).

10. The method according to Claim 9,
**characterised in that**
- the electrode (41) is positioned at a distance of 0.1 to 3 mm in relation to an inner side (42) of the preform (1).

11. The method according to any one of Claims 1 to 10,
**characterised in that**
- at least in some areas, the preform (1) is surrounded by an outer electrode (44) while the plasma treatment is carried out.

12. The method according to Claim 11,
**characterised in that**
- the outer electrode (44) is positioned at a distance of 0 to 2 mm in relation to an outer side (46) of the preform (1).

13. The method according to any one of Claims 1 to 12,
**characterised in that**
- the plasma is generated by a high voltage generator at a pulsed variable frequency.

14. The method according to any one of Claims 1 to 13,
**characterised in that**
- the preform (1) is moved along a transport route together with the sterilisation device (47).

15. A device for the sterilisation of injection moulded preforms made of a thermoplastic material, said preforms being provided for the manufacture of blow moulded containers,
**characterised in that**
- at least one sterilisation device (47) is provided with a plasma generator and that the preforms (2) are supplied to the blow moulding station after sterilisation.

16. The device according to Claim 15,
**characterised in that**
- the plasma generator comprises a rod electrode (41) for insertion into an interior region of the preform (1).

17. The device according to Claim 15 or 16,
**characterised in that**
- the plasma generator comprises an outer electrode (44) for enclosing the preform (1) at least in some areas.

18. The device according to Claim 17,
**characterised in that**
- the outer electrode (44) is formed like a beaker.

19. The device according to any one of Claims 15 to 16,
**characterised in that**
- the inner electrode is arranged at a distance of 0.1 to 3 mm in relation to an inner side (42) of the preform (1).

20. A device for blow moulding containers (2), the device comprising at least one blow station (3) for forming thermoplastic injection moulded preforms (1) into the containers (2), said blow station (3) being arranged on a bearing structure,
**characterised in that**
- at least one sterilisation device (47) that is provided with a plasma generator is arranged along a transport route of the preforms (1) and that the sterilisation device (47) is arranged upstream of the blow station (3) along the transport route.

## Revendications

1. Procédé de stérilisation de préformes (1) moulées par injection d'un matériau thermoplastique et destinées à la fabrication de récipients (2) moulés par soufflage, **caractérisé en ce que** la stérilisation est réalisée par application d'un plasma et que les préformes (2) sont soumises au moulage par soufflage après la stérilisation.

2. Procédé selon la revendication 1, **caractérisé en ce qu'**une surface intérieure des préformes (1) est stérilisée.

3. Procédé selon la revendication 1, **caractérisé en ce qu'**une surface extérieure des préformes (1) est stérilisée.

4. Procédé selon l'une des revendications 1 à 3, **caractérisé en ce que** le traitement au plasma est appliqué à pression ambiante.

5. Procédé selon l'une des revendications 1 à 3, **caractérisé en ce que** le traitement au plasma est appliqué à une pression supérieure à la pression ambiante.

6. Procédé selon l'une des revendications 1 à 5, **caractérisé en ce que** le traitement au plasma est appliqué en ayant recours à un gaz inerte.

7. Procédé selon l'une des revendications 1 à 6, **caractérisé en ce que** le traitement au plasma est appliqué avec une humidité relative du gaz comprise entre 15 et 95 % d'une absorption d'humidité maximale.

8. Procédé selon l'une des revendications 1 à 7, **caractérisé en ce que** le traitement au plasma est appliqué pendant un intervalle de temps de une à dix secondes.

9. Procédé selon l'une des revendications 1 à 8, **caractérisé en ce que** le traitement au plasma est appliqué en ayant recours à une électrode haute tension (41) positionnée dans un espace intérieur de la préforme (1).

10. Procédé selon la revendication 9, **caractérisé en ce que** l'électrode (41) est positionnée à une distance de 0,1 à 3 mm d'une face intérieure (42) de la préforme (1).

11. Procédé selon l'une des revendications 1 à 10, **caractérisé en ce que** la préforme (1) est en partie au moins entourée par une électrode extérieure (44) pendant l'application du traitement au plasma.

12. Procédé selon la revendication 11, **caractérisé en ce que** l'électrode extérieure (44) est positionnée à une distance de 0 à 2 mm d'une face extérieure (46) de la préforme (1).

13. Procédé selon l'une des revendications 1 à 12, **caractérisé en ce que** le plasma est généré par un générateur haute tension avec une fréquence variable pulsée.

14. Procédé selon l'une des revendications 1 à 13, **caractérisé en ce que** la préforme (1) est déplacée conjointement avec le dispositif de stérilisation (47) le long d'un parcours.

15. Dispositif pour stériliser des préformes moulées par injection d'un matériau thermoplastique et destinées à la fabrication de récipients moulés par soufflage, **caractérisé en ce qu'**au moins un dispositif de stérilisation (47) est équipé d'un générateur de plasma et **en ce que** les préformes (2) sont soumises au moulage par soufflage après la stérilisation.

16. Dispositif selon la revendication 15, **caractérisé en ce que** le générateur de plasma présente une électrode en forme de barre (41) destinée à être introduite dans un espace intérieur de la préforme (1).

17. Dispositif selon la revendication 15 ou 16, **caractérisé en ce que** le générateur de plasma présente une électrode extérieure (44) entourant en partie au moins la préforme (1).

18. Dispositif selon la revendication 17, **caractérisé en ce que** l'électrode extérieure (44) est réalisée en forme de godet.

19. Dispositif selon l'une des revendications 15 à 18, **caractérisé en ce que** l'électrode intérieure est disposée à une distance de 0,1 à 3 mm d'une face intérieure (42) de la préforme (1).

20. Dispositif de moulage par soufflage de récipients (2) qui présente, agencée sur une structure de support, au moins une station de soufflage (3) pour le formage des récipients (2) à partir de préformes (1) thermoplastiques moulées par injection, **caractérisé en ce que** le long d'un parcours des préformes (1) est agencé au moins un dispositif de stérilisation (47) muni d'un générateur de plasma et **en ce que**, le long du parcours, le dispositif de stérilisation (47) est placé devant la station de soufflage (3).
